(19) Europäisches Patentamt / European Patent Office / Office européen des brevets

(11) **EP 3 985 530 A1**

(12) **DEMANDE DE BREVET EUROPEEN**

(43) Date de publication:
**20.04.2022 Bulletin 2022/16**

(21) Numéro de dépôt: **21201998.8**

(22) Date de dépôt: **11.10.2021**

(51) Classification Internationale des Brevets (IPC):
*G06F 17/16* (2006.01)   *A61F 2/72* (2006.01)
*A61F 4/00* (2006.01)

(52) Classification Coopérative des Brevets (CPC):
**G06F 17/16; A61F 2/72; A61F 4/00**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **14.10.2020 FR 2010497**

(71) Demandeur: **Commissariat à l'énergie atomique et aux énergies alternatives**
**75015 Paris (FR)**

(72) Inventeurs:
• **MOLY, Alexandre**
  **38054 GRENOBLE CEDEX 09 (FR)**
• **AKSENOVA, Tetiana**
  **38054 GRENOBLE CEDEX 09 (FR)**
• **AKSENOV, Alexandre**
  **38054 GRENOBLE CEDEX 09 (FR)**

(74) Mandataire: **Brevalex**
**56, Boulevard de l'Embouchure**
**B.P. 27519**
**31075 Toulouse Cedex 2 (FR)**

(54) **MÉTHODE DE CALIBRATION EN LIGNE ET À FACTEUR D'OUBLI D'UNE INTERFACE NEURONALE DIRECTE À RÉGRESSION MULTIVARIÉE PÉNALISÉE**

(57)    La présente invention concerne une méthode de calibration en ligne d'une interface neuronale directe mettant en œuvre une régression REW-NPLS entre un tenseur de calibration de sortie et un tenseur de calibration d'entrée. La régression REW-NPLS comprend une décomposition itérative PARAFAC du tenseur de covariance croisée entre le tenseur de calibration d'entrée et le tenseur de calibration de sortie, chaque itération PARAFAC comprenant une séquence de $M$ étapes élémentaires ($240_1$, $240_1$,.. $240_M$) de minimisation d'une métrique selon la méthode des moindres carrés alternés, chaque étape élémentaire de minimisation portant sur un projecteur et considérant les autres comme constants, ladite métrique comportant un terme de pénalisation fonction de la norme de ce projecteur, les éléments de ce projecteur ne faisant pas l'objet d'une pénalisation lors d'une itération PARAFAC $f$ n'étant pas pénalisables lors des itérations PARAFAC suivantes. Ladite méthode de calibration permet d'obtenir un modèle prédictif dont les coefficients non nuls sont rares par blocs.

FIG.2

**Description**

**DOMAINE TECHNIQUE**

La présente invention concerne le domaine des interfaces neuronales directes encore dénommées BCI (*Brain Computer Interface*) ou BMI (*Brain Machine Interface*). Elle trouve notamment à s'appliquer à la commande neuronale directe d'une machine, telle qu'un exosquelette ou un ordinateur.

**ÉTAT DE LA TECHNIQUE ANTÉRIEURE**

Les interfaces neuronales directes utilisent les signaux électro-physiologiques émis par le cortex cérébral pour élaborer un signal de commande. Ces interfaces neuronales ont fait l'objet de nombreuses recherches notamment dans le but de restaurer une fonction motrice à un sujet paraplégique ou tétraplégique à l'aide d'une prothèse ou d'une orthèse motorisée.

Les interfaces neuronales peuvent être de nature invasive ou non invasive. Les interfaces neuronales invasives utilisent des électrodes intracorticales (c'est-à-dire implantées dans le cortex) ou des électrodes corticales (disposées à la surface du cortex) recueillant dans ce dernier cas des signaux électrocorticographiques (ECoG). Les interfaces neuronales non invasives utilisent des électrodes placées sur le cuir chevelu pour recueillir des signaux électroencéphalographiques (EEG). D'autres types de capteurs ont été également envisagés comme des capteurs magnétiques mesurant les champs magnétiques induits par l'activité électrique des neurones du cerveau. On parle alors de signaux magnétoencéphalographiques (MEG).

Les interfaces neuronales directes utilisent avantageusement des signaux de type ECoG, présentant l'avantage d'un bon compromis entre biocompatibilité (matrice d'électrodes implantées à la surface du cortex) et qualité des signaux recueillis.

Les signaux ECoG ainsi mesurés doivent être traités afin d'estimer la trajectoire du mouvement désiré par le sujet et en déduire les signaux de commande de l'ordinateur ou de la machine. Par exemple, lorsqu'il s'agit de commander un exosquelette, l'interface BCI estime la trajectoire du mouvement désiré à partir des signaux électro-physiologiques mesurés et en déduit les signaux de contrôle permettant à l'exosquelette de reproduire la trajectoire en question. De manière similaire, lorsqu'il s'agit de commander un ordinateur, l'interface BCI estime par exemple la trajectoire souhaitée d'un pointeur ou d'un curseur à partir des signaux électro-physiologiques et en déduit les signaux de commande du curseur/pointeur.

L'estimation de trajectoire, et plus précisément celle des paramètres cinématiques (position, vitesse, accélération), est encore dénommée décodage neuronal dans la littérature. Le décodage neuronal permet notamment de commander un mouvement (d'une prothèse ou d'un curseur) à partir de signaux ECoG.

L'estimation de trajectoire et le calcul des signaux de contrôle de l'exosquelette ou de l'effecteur, nécessite généralement une phase d'apprentissage ou de calibration préalable, dite *off-line*. Lors de cette phase, le sujet imagine, observe ou réalise un mouvement selon une trajectoire déterminée pendant un intervalle de calibration donné. Les signaux électrophysiologiques mesurés pendant cet intervalle sont exploités en relation avec cette trajectoire pour construire un modèle prédictif et plus précisément pour calculer les paramètres de ce modèle.

La validité du modèle prédictif est cependant limitée dans le temps en raison de la non-stationnarité des signaux neuronaux. Pour cette raison, il est nécessaire de procéder à une calibration en ligne (*on-line*) du modèle prédictif, c'est-à-dire au fur et à mesure que les signaux neuronaux sont observés et la commande appliquée.

Différentes méthodes de calibration en ligne d'une interface neuronale ont été décrites dans l'état de la technique. Toutefois, ces méthodes de calibration en ligne peuvent nécessiter la manipulation d'une quantité considérable de données résultant de la juxtaposition de données d'étapes de calibration antérieures et celles d'une nouvelle étape de calibration.

Une méthode de calibration *on-line* d'une interface BCI a été décrite dans l'article de A. Eliseyev et al. intitulé « Recursive exponentially weighted N-way Partial Least Squares régression with recursive validation of hyper-parameters in Brain-Computer Interface applications » publié dans Scientific Reports, vol. 7, n° 1, p. 16281, nov. 2017 ainsi que dans la demande de brevet FR-A-3 061 318. Cette méthode sera désignée par la suite sous l'acronyme REW-NPLS (*Recursive Exponentially Weighted N-way Partial Least Squares*).

D'autre part, différentes variantes de méthode de calibration *off-line* ont été proposées dans la littérature, dans le but d'améliorer la robustesse de la prédiction et d'accroître la rareté (*sparsity*) des coefficients non nuls dans le tenseur du modèle prédictif. En particulier, la rareté des coefficients non nuls dans le mode spatial de ce tenseur permet de ne sélectionner que certaines électrodes pertinentes pour la prédiction de la trajectoire et le calcul de la commande. Une telle variante utilisant une régression multivoie pénalisée dans un tel but a été décrite dans l'article de A. Eliseyev et al. intitulé « L1-penalized N-way PLS for subset of electrodes selection in BCI experiments » publié dans J. Neural Eng. vol. 9, n° 4, p. 045010 ainsi que dans la demande de brevet FR-A-3 046 471.

Toutefois, une telle méthode de calibration utilisant une régression multivoie pénalisée ne peut être mise en œuvre en ligne en raison de la quantité importante de calculs qu'elle requiert.

L'objet de la présente invention est par conséquent de proposer une méthode de calibration en ligne d'une interface neuronale directe qui puisse également accroître la rareté des coefficients non nuls du tenseur de prédiction, notamment de manière à pouvoir ne sélectionner à chaque fenêtre d'observation qu'un sous-ensemble pertinent d'électrodes.

## EXPOSÉ DE L'INVENTION

La présente invention est définie par une méthode de calibration en ligne d'une interface neuronale directe destinée à recevoir une pluralité de signaux électrophysiologiques pendant une pluralité de fenêtres d'observation associées à des instants d'observation, pour former un tenseur d'entrée et en déduire, au moyen d'un modèle prédictif, un tenseur de sortie fournissant des signaux de commande destinés à commander un ou plusieurs effecteurs, ladite calibration est effectuée lors d'une pluralité d'étapes de calibration, chaque étape de calibration u étant réalisée à partir de données de calibration d'entrée, représentées par un tenseur de calibration d'entrée, $\underline{\mathbf{X}}_u \in \mathbb{R}^{(I_1 \times \ldots \times I_N)}$, et de données de calibration de sortie représentées par un tenseur de calibration de sortie $\underline{\mathbf{Y}}_u \in \mathbb{R}^{(J_1 \times \ldots \times J_M)}$, ladite étape de calibration mettant en œuvre une régression REW-NPLS comportant :

le calcul d'un tenseur de covariance $\underline{\mathbf{XX}}_u$ à partir d'un tenseur de covariance $\underline{\mathbf{XX}}_{u-1}$ obtenu lors d'une étape de calibration précédente et du tenseur $\underline{\mathbf{X}}_u$, ainsi que le calcul d'un tenseur de covariance croisée $\underline{\mathbf{XY}}_u$ à partir d'un tenseur de covariance croisée $\underline{\mathbf{XY}}_{u-1}$ obtenu lors de ladite étape de calibration précédente et des tenseurs $\underline{\mathbf{X}}_u$ et $\underline{\mathbf{Y}}_u$ ; une décomposition PARAFAC itérative du tenseur de covariance croisée $\underline{\mathbf{XY}}_u$, chaque itération PARAFAC étant associée à une dimension $f$ de l'espace des variables latentes sur lequel est effectuée ladite régression, chaque itération fournissant une pluralité de projecteurs $w^i_f$, $i = 1,..,M$ de tailles respectives $I_1, I_2, ..I_M$ et un modèle prédictif défini par un tenseur de coefficients de prédiction $\underline{\mathbf{B}}_u^f \in \mathbb{R}^{(I_1 \times \ldots \times I_N) \times (J_1 \times \ldots \times J_M)}$ et un tenseur de biais $\underline{\boldsymbol{\beta}}_u^f \in \mathbb{R}^{(J_1 \times \ldots \times J_M)}$.

Ladite méthode de calibration est originale en ce que chaque itération PARAFAC comprend une séquence de $M$ étapes élémentaires de minimisation d'une métrique selon la méthode des moindres carrés alternés, chaque étape élémentaire de minimisation portant sur un projecteur et considérant les autres comme constants, ladite métrique comportant un terme de pénalisation fonction de la norme de ce projecteur, les éléments de ce projecteur ne faisant pas l'objet d'une pénalisation lors d'une itération PARAFAC $f$ n'étant pas pénalisables lors des itérations PARAFAC suivantes.

Avantageusement, une étape élémentaire de minimisation portant sur un projecteur $w^i_f$ vise à minimiser la métrique pénalisée

$$\min_{\tilde{\mathbf{w}}^i_f} \left( \left\| \underline{\mathbf{XY}}_{u(i)} - \tilde{\mathbf{w}}^i_f \left( \mathbf{w}^M_f \otimes \ldots \otimes \mathbf{w}^{i+1}_f \otimes \mathbf{w}^{i-1}_f \otimes \ldots \otimes \mathbf{w}^1_f \right)^T \right\|^2 + \lambda_i \left\| \tilde{\mathbf{w}}^i_f \right\|_{q, \Omega_{i,f}} \right)$$

où $\tilde{\mathbf{w}}^i_f$ est un projecteur non normalisé et $\mathbf{w}^m_f = \dfrac{\tilde{\mathbf{w}}^m_f}{\left\| \tilde{\mathbf{w}}^m_f \right\|}$, $m = 1, ..., M$; $m \neq i$ sont des projecteurs normalisés, $\underline{\mathbf{XY}}_{u(i)}$ est une matrice de taille $I_i \times \prod\limits_{\substack{n=1 \\ n \neq i}}^{N} I_n$ obtenue par déploiement du tenseur de covariance croisée $\underline{\mathbf{XY}}_u$ en prenant le mode $i$ comme référence, $\otimes$ est le produit de Kronecker, $\lambda_i$ est un coefficient strictement positif, $\left\| \mathbf{w} \right\|_{q, \Omega_{i,f}}$, $q = 0, \dfrac{1}{2}$, 1 est une norme définie par

$$\left\| \mathbf{w}^i_f \right\|_{0, \Omega_{i,f}} = \sum_{k \in \Omega_{i,f}} \left( 1 - \delta_{0, w^i_{f,k}} \right), \quad \left\| \mathbf{w}^i_f \right\|_{1/2, \Omega_{i,f}} = \sum_{k \in \Omega_{i,f}} \sqrt{\left| w^i_{f,k} \right|}, \quad \left\| \mathbf{w}^i_f \right\|_{1, \Omega_{i,f}} = \sum_{k \in \Omega_{i,f}} \left| w^i_{f,k} \right|$$

où $w^i_{f,k}$ est le $k^{ème}$ élé-

ment du projecteur $\mathbf{w}^i_f$, $\delta$ est le symbole de Kronecker, et $\Omega_{i,f}$ est l'ensemble des indices correspondant aux éléments pénalisables du projecteur $\mathbf{w}^i_f$ à l'itération PARAFAC $f$.

Alternativement, une étape élémentaire de minimisation portant sur un projecteur $\mathbf{w}^i_f$ vise à minimiser les

$$\min_{w^i_{f,k}} \left( \left\| \mathbf{z}^k_i - w^i_{f,k} \left( \left( \mathbf{w}^M_f \otimes ... \mathbf{w}^{i+1}_f \otimes \mathbf{w}^{i-1}_f \otimes ... \otimes \mathbf{w}^1_f \right)^T \right)^T \right\|^2 + \lambda_i g_q \left( w^i_{f,k} \right) \right)$$

métriques pénalisées , $k=1,...,I_i$ où

$$I_i \times \prod_{\substack{n=1 \\ n \neq i}}^{N} I_n$$

$\mathbf{z}^k_i$ est le kème vecteur colonne de la matrice $\underline{\mathbf{XY}}_{u(i)}$ de taille obtenue par déploiement du tenseur de covariance croisée $\underline{\mathbf{XY}}_u$ en prenant le mode i comme référence, $\otimes$ est le produit de Kronecker, $\lambda_i$ est un coefficient

strictement positif, et Les fonctions $g_q$, $q = 0, \dfrac{1}{2}, 1$ sont définies par :

$$g_0 \left( w^i_{f,k} \right) = 1 - \delta_{0, w^i_{f,k}} \quad \text{si} \quad k \in \Omega_{i,f} \quad \text{et} \quad g_0 \left( w^i_{f,k} \right) = 0$$

sinon

$$g_{1/2} \left( w^i_{f,k} \right) = \sqrt{\left| w^i_{f,k} \right|} \quad \text{si} \quad k \in \Omega_{i,f} \quad \text{et} \quad g_{1/2} \left( w^i_{f,k} \right) = 0$$

sinon

$$g_1 \left( w^i_{f,k} \right) = \left| w^i_{f,k} \right| \quad \text{si} \quad k \in \Omega_{i,f} \quad \text{et} \quad g_1 \left( w^i_{f,k} \right) = 0 \quad \text{sinon}$$

sinon

où $\Omega_{i,f}$ est l'ensemble des indices $k$ correspondant aux éléments $w^i_{f,k}$ pénalisables du projecteur $\mathbf{w}^i_f$ à l'itération PARAFAC $f$.

Dans un cas particulier de réalisation, $q=0$ et les éléments $w^i_{f,k}$ résultant de la minimisation sont donnés par :

$$\left( w^i_{f,k} \right)_{L0} = 0 \quad \text{si} \quad k \in \Omega_{i,f} \quad \text{et} \quad \left| \left( w^i_{f,k} \right)_{LS} \right| \leq Th^i_{L0}$$

$$\left( w^i_{f,k} \right)_{L0} = \left( w^i_{f,k} \right)_{LS}$$

sinon où $\left( w^i_{f,k} \right)_{LS}$ représente la solution de minimisation au sens des moindres carrés :

$$\left( w^i_{f,k} \right)_{LS} = \frac{\mathbf{z}^k_i \left( \mathbf{w}^M_f \otimes ... \mathbf{w}^{i+1}_f \otimes \mathbf{w}^{i-1}_f \otimes ... \otimes \mathbf{w}^1_f \right)}{\left\| \mathbf{w}^M_f \otimes ... \mathbf{w}^{i+1}_f \otimes \mathbf{w}^{i-1}_f \otimes ... \otimes \mathbf{w}^1_f \right\|^2}$$

et que le seuil $Th_{L0}^i$ est donné par

$$Th_{L0}^i = \frac{\sqrt{\lambda_i}}{\left\| \mathbf{w}_f^M \otimes ... \mathbf{w}_f^{i+1} \otimes \mathbf{w}_f^{i-1} \otimes ... \otimes \mathbf{w}_f^1 \right\|}$$ .

Dans un second cas particulier de réalisation, $q = \dfrac{1}{2}$ et les éléments $w_{f,k}^i$ résultant de la minimisation sont données par :

$$\left( w_{f,k}^i \right)_{L0} = 0 \quad \text{si} \quad k \in \Omega_{i,f} \quad \text{et} \quad \left| \left( w_{f,k}^i \right)_{LS} \right| \le Th_{L0,5}^i$$

$$\left( w_{f,k}^i \right)_{L0} = \arg\min\left( h_{0,5}(0), h_{0,5}\left( B^i . \left( w_{f,k}^i \right)_{LS} \right) \right) \quad \text{si} \quad k \in \Omega_{i,f} \quad \text{et} \quad \left| \left( w_{f,k}^i \right)_{LS} \right| > Th_{L0,5}^i$$

$$\left( w_{f,k}^i \right)_{L0} = \left( w_{f,k}^i \right)_{LS}$$

sinon

où $h_{0,5}\left( w_{f,k}^i \right) = \left\| \mathbf{z}_1^k - w_{f,k}^i \left( \mathbf{w}_f^3 \otimes \mathbf{w}_f^2 \right)^T \right\|^2 + \lambda_i \sqrt{\left| w_{f,k}^i \right|}$ et où $\left( w_{f,k}^i \right)_{LS}$ représente la solution de minimisation au sens des moindres carrés :

$$\left( w_{f,k}^i \right)_{LS} = \frac{\mathbf{z}_i^k \left( \mathbf{w}_f^M \otimes ... \mathbf{w}_f^{i+1} \otimes \mathbf{w}_f^{i-1} \otimes ... \otimes \mathbf{w}_f^1 \right)}{\left\| \mathbf{w}_f^M \otimes ... \mathbf{w}_f^{i+1} \otimes \mathbf{w}_f^{i-1} \otimes ... \otimes \mathbf{w}_f^1 \right\|^2}$$

$$Th_{L0,5}^i = \frac{3}{4}\left( \frac{\lambda_i}{\left\| \mathbf{w}_f^M \otimes ... \mathbf{w}_f^{i+1} \otimes \mathbf{w}_f^{i-1} \otimes ... \otimes \mathbf{w}_f^1 \right\|^2} \right)^{\frac{2}{3}}$$

2 et le seuil $Th_{L0,5}^i$ est donné par                                                                                                           .

Dans un troisième cas particulier de réalisation, $q = 1$ et les éléments $w_{f,k}^i$ résultant de la minimisation sont données par :

$$\left( w_{f,k}^i \right)_{L1} = 0 \quad \text{si} \quad k \in \Omega_{i,f} \quad \text{et} \quad \left| \left( w_{f,k}^i \right)_{LS} \right| \le Th_{L1}^i$$

$$\left( w_{f,k}^i \right)_{L1} = \text{sgn}\left( \left( w_{f,k}^i \right)_{LS} \right)\left( \left| \left( w_{f,k}^i \right)_{LS} \right| - Th_{L1}^i \right) \quad \text{si} \quad k \in \Omega_{i,f} \quad \text{et} \quad \left| \left( w_{f,k}^i \right)_{LS} \right| > Th_{L1}^i$$

$$\left( w_{f,k}^i \right)_{L0} = \left( w_{f,k}^i \right)_{LS}$$

sinon

où $\left(w_{f,k}^{i}\right)_{LS}$ représente la solution de minimisation au sens des moindres carrés :

$$\left(w_{f,k}^{i}\right)_{LS} = \frac{\mathbf{z}_i^k \left(\mathbf{w}_f^M \otimes ... \mathbf{w}_f^{i+1} \otimes \mathbf{w}_f^{i-1} \otimes ... \otimes \mathbf{w}_f^1\right)}{\left\|\mathbf{w}_f^M \otimes ... \mathbf{w}_f^{i+1} \otimes \mathbf{w}_f^{i-1} \otimes ... \otimes \mathbf{w}_f^1\right\|^2}$$

$$Th_{L1}^i = \frac{\lambda_i}{\left\|\mathbf{w}_f^M \otimes ... \mathbf{w}_f^{i+1} \otimes \mathbf{w}_f^{i-1} \otimes ... \otimes \mathbf{w}_f^1\right\|}$$

et le seuil $Th_{L1}^i$ est donné par

Typiquement, ladite méthode de calibration en ligne peut s'appliquer à des signaux électrophysiologiques constitués de signaux ECoG.

## BRÈVE DESCRIPTION DES DESSINS

D'autres caractéristiques et avantages de l'invention apparaîtront à la lecture d'un mode de réalisation préférentiel de l'invention, décrit en référence aux figures jointes parmi lesquelles :

La Fig. 1A représente de manière schématique la décomposition d'un tenseur par analyse factorielle parallèle (PARAFAC) ;
La Fig. 1B représente de manière schématique le développement d'un tenseur par rapport à un mode ;
La Fig. 1C représente de manière schématique une décomposition utilisant une méthode des moindres carrés alternés (ALS) ;
La Fig. 2 représente de manière schématique l'ordinogramme d'une méthode de calibration récursive d'une interface neuronale directe à régression multivariée pénalisée, selon un mode de réalisation de la présente invention ;
La Fig. 3 représente de manière schématique le mécanisme de mise à jour du modèle prédictif dans la méthode de calibration de la Fig. 2.

## EXPOSÉ DÉTAILLÉ DE MODES DE RÉALISATION PARTICULIERS

On considérera dans la suite une interface neuronale directe à décodage continu.

Les signaux électrophysiologiques issus des différentes électrodes sont échantillonnés et rassemblés par blocs de données, chaque bloc correspondant à une fenêtre glissante d'observation de largeur $\Delta T$. Chaque fenêtre d'observation est définie par un instant d'observation (*epoch*) auquel démarre la fenêtre en question.

Les signaux électrophysiologiques peuvent faire l'objet d'un prétraitement. Ce prétraitement peut notamment comporter une suppression de la moyenne prise sur l'ensemble des électrodes, puis une analyse temps-fréquence est effectuée sur chacune des fenêtres d'observation.

L'analyse temps-fréquence est basée sur une décomposition en ondelettes, notamment en ondelettes de Morlet. L'homme du métier comprendra toutefois que d'autres type d'analyse temps-fréquence pourront être envisagés par l'homme du métier.

Ces résultats de l'analyse temps-fréquence peuvent en outre faire l'objet d'un lissage fréquentiel ou d'une décimation.

Ainsi, à chaque fenêtre d'observation, ou instant d'observation t, est associé un tenseur d'ordre 3 de données d'observation, d'où la génération d'un tenseur d'entrée d'ordre 4: le premier mode correspond aux fenêtres d'observation successives, le second mode correspond à l'espace, autrement dit aux capteurs, le troisième mode correspond au temps au sein d'une fenêtre d'observation, autrement dit aux positions des ondelettes, et le quatrième mode correspond à la fréquence, autrement dit au nombre de bandes de fréquence utilisées pour la décomposition en ondelettes sur une fenêtre d'observation.

De manière plus générale, le tenseur d'entrée (ou tenseur d'observation) sera d'ordre $N$, le premier mode étant dans tous les cas celui relatif aux instants d'observation (*epochs*). Le tenseur d'entrée (ou tenseur d'observation) est noté **X** et est de dimension $I_1 \times ... \times I_N$.

De la même façon, la trajectoire du mouvement imaginé, observé ou réalisé est décrite par un tenseur de sortie (ou tenseur de commande) d'ordre $M$, noté Y, de dimension $J_1 \times ... \times J_M$, dont le premier mode correspond aux instants successifs auxquels s'appliqueront les commandes (en règle générale, ce premier mode correspond également aux fenêtres d'observation), les autres modes correspondant aux commandes de différents effecteurs ou aux différents degrés de liberté d'un robot multi-axe.

Plus précisément, le tenseur de sortie fournit des blocs de données de commande, chacun des blocs permettant de générer les signaux de commande relatifs aux différents effecteurs ou degrés de liberté. Ainsi, on comprendra que la dimension de chaque bloc de données pourra dépendre du cas d'usage envisagé et notamment du nombre de degrés de liberté de l'effecteur.

On notera dans la suite $\underline{\mathbf{X}}_t$ le tenseur d'observation à l'instant $t$. Ce tenseur est par conséquent d'ordre $N$ et de dimension $I_1 \times ... \times I_N$. Il prend ses valeurs dans un espace $\underline{X} \subset \mathbb{R}^{I_1 \times ... \times I_N}$ où $\mathbb{R}$ est l'ensemble de réels. De manière similaire, on notera $\underline{\mathbf{Y}}_t$ le tenseur de commande à l'instant $t$. Ce tenseur de sortie est d'ordre $M$ et de dimension $J_1 \times ... \times J_M$. Il prend ses valeurs dans un espace $\underline{Y} \subset \mathbb{R}^{J_1 \times ... \times J_M}$ .

La calibration ou, de manière équivalente, le mécanisme de mise à jour du modèle prédictif selon l'algorithme REW-NPLS (*Recursive Exponentially Weigthed N-way Partial Least Squares*) est rappelé ci-après.

Si l'on note $\{\underline{\mathbf{X}}_u, \underline{\mathbf{Y}}_u\}$ les blocs de données de calibration *(training data set)* lors de l'itération $u$, l'idée de base de l'algorithme REW-NPLS est de mettre à jour le modèle prédictif à partir des tenseurs de covariance et de covariance croisée obtenus à l'étape de calibration précédente, respectivement notés $\underline{\mathbf{XX}}_{u-1}$ et $\underline{\mathbf{XY}}_{u-1}$ . Ces deux tenseurs permettent d'obtenir une représentation condensée du modèle prédictif à l'étape précédente, sans qu'il soit besoin de stocker l'historique des blocs de données de calibration précédents : $\{\underline{\mathbf{X}}_1, \underline{\mathbf{Y}}_1\}, \{\underline{\mathbf{X}}_2, \underline{\mathbf{Y}}_2\}, ... \{\underline{\mathbf{X}}_{u-1}, \underline{\mathbf{Y}}_{u-1}\}$.

Plus précisément, les tenseurs de covariance et de covariance croisée sont mis à jour au moyen des relations de récurrence :

$$\underline{\mathbf{XX}}_u = \gamma \underline{\mathbf{XX}}_{u-1} + \underline{\mathbf{X}}_u \times_1 \underline{\mathbf{X}}_u \tag{1-1}$$

$$\underline{\mathbf{XY}}_u = \gamma \underline{\mathbf{XY}}_{u-1} + \underline{\mathbf{X}}_u \times_1 \underline{\mathbf{Y}}_u \tag{1-2}$$

où $\times_1$ désigne le produit de tenseur de mode 1 et $\gamma$ est un coefficient d'oubli avec $0 < \gamma < 1$.

On comprend ainsi qu'une période de calibration courante, réalisée lors de l'itération $u$ tient compte d'une période de calibration précédente réalisée lors de l'itération $u$-1 et des tenseurs des données d'observation de la période de calibration courante, l'historique étant pondéré par le coefficient d'oubli $\gamma$ avec $0 < \gamma < 1$.

Cette opération de mise à jour des tenseurs de covariance est effectuée de manière itérative sur les périodes de calibration.

Pour chaque mise à jour, le modèle prédictif est ensuite obtenu de manière itérative au moyen d'une décomposition PARAFAC (généralisation au cas tensoriel d'une décomposition en valeurs singulières) du tenseur de covariance croisée, $\underline{\mathbf{XY}}_u$. On rappelle que, d'une manière générale, une décomposition PARAFAC d'un tenseur $\underline{\mathbf{Z}}$ d'ordre $M$, soit $\underline{\mathbf{Z}} \in \mathbb{R}^{I_1 \times I_2 \times ... \times I_M}$ permet de représenter ce tenseur sous la forme d'une combinaison linéaire de produits extérieurs de vecteurs (tenseurs d'ordre 1) à un tenseur résiduel près :

$$\underline{\mathbf{Z}} = \sum_{r=1}^{R} \theta_r \mathbf{w}_r^1 \circ \mathbf{w}_r^2 \circ ... \circ \mathbf{w}_r^M + \underline{\mathbf{E}} \tag{2}$$

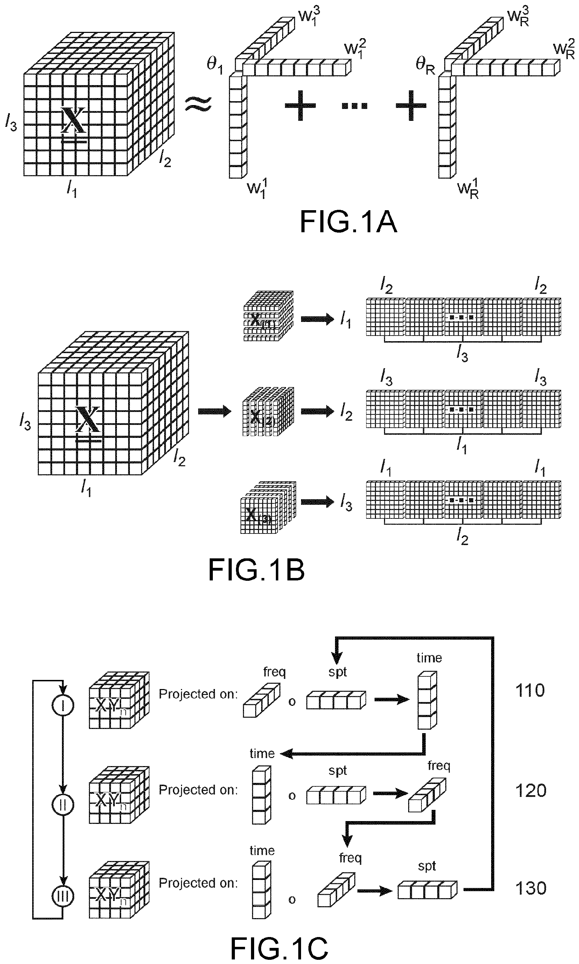

avec $\left\| \mathbf{w}_r^m \right\| = 1$ , $m = 1,..,M$ où $\circ$ est le produit extérieur et $\underline{\mathbf{E}}$ est un tenseur résiduel et r est l'indice de l'itération PARAFAC et R est un entier donné. Une décomposition PARFAC d'un tenseur $\underline{\mathbf{Z}}$ a été représenté schématiquement en Fig. 1A.

Dans le cas d'une étape de calibration REW-NPLS, chaque itération PARAFAC ne porte que sur une décomposition élémentaire, c'est-à-dire R =1. Elle permet d'extraire du tenseur $\underline{\mathbf{XY}}_u$ un ensemble de vecteurs de projection, encore dénommés projecteurs, $\mathbf{w}_f^1, ..., \mathbf{w}_f^M$ , de tailles respectives $I_1, I_2, .., I_M$ où le numéro d'itération $f$ donne la dimension d'un espace de variables latentes sur lequel est projet le tenseur d'entrée $\underline{\mathbf{X}}_u$ .

Afin de simplifier la présentation et sans préjudice de généralisation à un tenseur d'ordre quelconque, nous supposerons dans la suite que l'ordre du tenseur $\underline{\mathbf{XY}}_u$ est égal à 3 ($M = 3$), c'est-à-dire $\underline{\mathbf{XY}}_u \in \mathbb{R}^{I_1 \times I_2 \times I_3}$ avec $||\underline{\mathbf{XY}}_u|| = 1$. Par exemple, le premier mode correspond à l'époque, le second à la fréquence et le troisième à l'espace (c'est-à-dire aux électrodes).

A l'itération $f$ de la décomposition PARAFAC, on recherche les vecteurs de projection $\mathbf{w}_f^1$, $\mathbf{w}_f^2$, $\mathbf{w}_f^3$ qui vérifient :

$$\min_{\underline{\mathbf{XY}}_u} \left\| \underline{\mathbf{XY}}_u - \widehat{\underline{\mathbf{XY}}_u} \right\|^2 \quad \text{avec} \quad \widehat{\underline{\mathbf{XY}}_u} = \theta_f \mathbf{w}_f^1 \circ \mathbf{w}_f^2 \circ \mathbf{w}_f^3 \quad \text{et} \quad \left\|\mathbf{w}_f^1\right\| = \left\|\mathbf{w}_f^2\right\| = \left\|\mathbf{w}_f^3\right\| = 1$$

$$(3)$$

Ce problème de minimisation peut être résolu par une méthode des moindres carrés alternés dans laquelle on effectue séquentiellement une minimisation pour chacun des vecteurs de la décomposition, soit :

$$\min_{\mathbf{w}_f^1} \left\| \underline{\mathbf{XY}}_{u(1)} - \mathbf{w}_f^1 \left( \mathbf{w}_f^3 \otimes \mathbf{w}_f^2 \right)^T \right\|^2 \qquad (4\text{-}1)$$

$$\min_{\mathbf{w}_f^2} \left\| \underline{\mathbf{XY}}_{u(2)} - \mathbf{w}_f^2 \left( \mathbf{w}_f^3 \otimes \mathbf{w}_f^1 \right)^T \right\|^2 \qquad (4\text{-}2)$$

$$\min_{\mathbf{w}_f^3} \left\| \underline{\mathbf{XY}}_{u(3)} - \mathbf{w}_f^3 \left( \mathbf{w}_f^2 \otimes \mathbf{w}_f^1 \right)^T \right\|^2 \qquad (4\text{-}3)$$

où $\otimes$ est le produit de Kronecker et $\underline{\mathbf{Z}}_{(n)}$ est une matrice obtenue par déploiement du tenseur $\underline{\mathbf{Z}}$ en prenant le mode $n$ comme mode de référence (*tensor unfolding or flattening along mode n*). Par exemple, la matrice $\underline{\mathbf{Z}}_{(1)}$ est définie par $\left( \mathbf{z}_1^1 \Big| \ldots \Big| \mathbf{z}_1^{I_1} \right) \in \mathbb{R}^{I_1 \times I_2 I_3}$ où les $\mathbf{z}_1^j$ sont les colonnes de la matrice $\underline{\mathbf{Z}}_{(1)}$.

Les opérations de minimisation (4-1), (4-2) et (4-3) sont répétées jusqu'à la convergence. Les solutions de minimisation au sens des moindres carrés (LS) pour chacune de ces opérations sont données par les projecteurs :

$$\mathbf{w}_f^1 = \frac{\underline{\mathbf{XY}}_{u(1)} \left( \mathbf{w}_f^3 \otimes \mathbf{w}_f^2 \right)}{\left\| \mathbf{w}_f^3 \otimes \mathbf{w}_f^2 \right\|^2} \qquad (5\text{-}1)$$

$$\mathbf{w}_f^2 = \frac{\underline{\mathbf{XY}}_{u(2)} \left( \mathbf{w}_f^3 \otimes \mathbf{w}_f^1 \right)}{\left\| \mathbf{w}_f^3 \otimes \mathbf{w}_f^1 \right\|^2} \qquad (5\text{-}2)$$

$$\mathbf{w}_f^3 = \frac{\underline{\mathbf{XY}}_{u(3)} \left( \mathbf{w}_f^2 \otimes \mathbf{w}_f^1 \right)}{\left\| \mathbf{w}_f^2 \otimes \mathbf{w}_f^1 \right\|^2} \qquad (5\text{-}3)$$

Chaque itération $f$ de la décomposition PARAFAC fournit une pluralité $M$ de projecteurs $\mathbf{w}_f^1, ..., \mathbf{w}_f^M$ et un tenseur de coefficients de prédiction, ou modèle prédictif de l'interface neuronale directe, défini par un tenseur de coefficients de prédiction $\underline{\mathbf{B}}_u^f \in \mathbb{R}^{(I_1 \times ... \times I_N) \times (J_1 \times ... \times J_M)}$ et un tenseur de biais $\underline{\boldsymbol{\beta}}_u^f \in \mathbb{R}^{(J_1 \times ... \times J_M)}$ permettant prédire le tenseur de commande à partir du tenseur d'observation. On obtient ainsi une pluralité de modèles, $\underline{\mathbf{B}}_u^f$, $f = 1,...,F$ correspondant à différentes dimensions successives de l'espace des variables latentes.

On détermine ensuite, à l'étape de calibration suivante, u+l, parmi $\underline{\mathbf{B}}_u^f$, $f = 1,...,F$, le modèle prédictif $\underline{\mathbf{B}}_u^{f^*}$ correspondant à l'erreur de prédiction la plus faible où l'on a noté $f^*$ l'indice $f$ correspondant à cette plus faible erreur de prédiction. Cette détermination du modèle prédictif à l'étape de calibration suivante est désignée dans la littérature relative sous le terme de validation récursive.

La Fig. 1C représente de manière schématique une décomposition utilisant une méthode des moindres carrés alternés (ALS).

La décomposition par les moindres carrés alternés a été illustrée pour une étape de calibration REW-NPLS et une itération $f$ PARAFAC .

A l'étape 110, on estime le vecteur $\mathbf{w}_f^1$ (correspondant au mode époque) à partir de la matrice $\underline{\mathbf{XY}}_{u(1)}$ et des vecteurs $\mathbf{w}_f^2$ et $\mathbf{w}_f^3$ courants. Cette opération peut être considérée comme une projection des vecteurs colonnes de la matrice $\underline{\mathbf{XY}}_{u(1)}$ sur l'espace tensoriel généré par les modes 2 et 3.

A l'étape 120, on estime le vecteur $\mathbf{w}_f^2$ (correspondant au mode fréquence ) à partir de la matrice $\underline{\mathbf{XY}}_{u(2)}$ et des vecteurs $\mathbf{w}_f^1$ et $\mathbf{w}_f^3$ courants. Cette opération peut être considérée comme une projection des vecteurs colonnes de la matrice $\underline{\mathbf{XY}}_{u(2)}$ sur l'espace tensoriel généré par les modes 1 et 3.

A l'étape 130, on estime le vecteur $\mathbf{w}_f^3$ (correspondant au mode spatial) à partir de la matrice $\underline{\mathbf{XY}}_{u(3)}$ et des vecteurs $\mathbf{w}_f^1$ et $\mathbf{w}_f^2$ courants. Cette opération peut être considérée comme une projection des vecteurs colonnes de la matrice $\underline{\mathbf{XY}}_{u(3)}$ sur l'espace tensoriel généré par les modes 1 et 2.

Les étapes 110, 120, 130 sont itérées jusqu'à ce qu'un critère de convergence soit rempli, par exemple lorsque la somme des écarts entre les vecteurs $\mathbf{w}_f^j$ de deux itérations ALS successives est inférieure à une valeur de seuil prédéterminée.

L'idée à la base de la présente invention est d'effectuer une pénalisation par tranche à chaque itération de l'algorithme PARAFAC, une pénalisation par tranche se traduisant par une élimination des éléments relatifs à une valeur d'indice dans le tenseur du modèle de prédiction. Les éléments ne faisant pas l'objet d'une pénalisation lors d'une itération $f$ ne sont plus pénalisables pour les itérations suivantes, $f + 1, f + 2,...,F$. En revanche les éléments pénalisés lors d'une itération sont pris en compte lors de la décomposition PARAFAC de l'itération suivante. On promeut ainsi une rareté par blocs (*blockwise sparsity*) des éléments non nuls dans le tenseur du modèle de prédiction, ce qui simplifie considérablement le calcul de la commande.

Plus précisément, lors d'une itération $f$ de la décomposition PARAFAC, la minimisation des écarts quadratiques selon (4-1), (4-2) et (4-3) dans la méthode des moindres carrés alternés est remplacée par une minimisation prenant en compte un terme de pénalisation favorisant cette rareté, à savoir :

$$\min_{\tilde{\mathbf{w}}_f^1}\left( \left\| \underline{\mathbf{XY}}_{u(1)} - \tilde{\mathbf{w}}_f^1 \left( \mathbf{w}_f^3 \otimes \mathbf{w}_f^2 \right)^T \right\|^2 + \lambda_1 \left\| \tilde{\mathbf{w}}_f^1 \right\|_{q, \Omega_{1,f}} \right) \tag{6-1}$$

$$\min_{\tilde{\mathbf{w}}_f^2}\left(\left\|\underline{\mathbf{XY}}_{u(2)} - \tilde{\mathbf{w}}_f^2\left(\mathbf{w}_f^3 \otimes \mathbf{w}_f^1\right)^T\right\|^2 + \lambda_2 \left\|\tilde{\mathbf{w}}_f^2\right\|_{q,\Omega_{2,f}}\right) \tag{6-2}$$

$$\min_{\tilde{\mathbf{w}}_f^3}\left(\left\|\underline{\mathbf{XY}}_{u(3)} - \tilde{\mathbf{w}}_f^3\left(\mathbf{w}_f^2 \otimes \mathbf{w}_f^1\right)^T\right\|^2 + \lambda_3 \left\|\tilde{\mathbf{w}}_f^3\right\|_{q,\Omega_{3,f}}\right) \tag{6-3}$$

où $\tilde{\mathbf{w}}_f^1$, $\tilde{\mathbf{w}}_f^2$ et $\tilde{\mathbf{w}}_f^3$ sont des projecteurs non normalisés et $\mathbf{w}_f^1 = \dfrac{\tilde{\mathbf{w}}_f^1}{\left\|\tilde{\mathbf{w}}_f^1\right\|}$, $\mathbf{w}_f^2 = \dfrac{\tilde{\mathbf{w}}_f^2}{\left\|\tilde{\mathbf{w}}_f^2\right\|}$, $\mathbf{w}_f^3 = \dfrac{\tilde{\mathbf{w}}_f^3}{\left\|\tilde{\mathbf{w}}_f^3\right\|}$ sont les projecteurs normalisés correspondants et $\lambda_1$, $\lambda_2$, $\lambda_3$ sont des coefficients strictement positifs et inférieurs à 1. On notera que du fait de la présence des termes de pénalisation dans les expressions (6-1), (6-2) et (6-3), la minimisation ne peut plus être réalisée sur des vecteurs de projection normalisés comme dans la méthode ALS classique. Les normes $\left\|\mathbf{w}_f^i\right\|_{q,\Omega_i}$ $q = 0, \dfrac{1}{2}, 1$ sont définies par :

$$\left\|\mathbf{w}_f^i\right\|_{0,\Omega_{i,f},} = \sum_{k \in \Omega_{i,f}}\left(1 - \delta_{0,w_{f,k}^i}\right) \tag{7-1}$$

$$\left\|\mathbf{w}_f^i\right\|_{1/2,\Omega_{i,f}} = \sum_{k \in \Omega_{i,f}}\sqrt{\left|w_{f,k}^i\right|} \tag{7-2}$$

$$\left\|\mathbf{w}_f^i\right\|_{1,\Omega_{i,f}} = \sum_{k \in \Omega_{i,f}}\left|w_{f,k}^i\right| \tag{7-3}$$

$w_{f,k}^i$ est le $k^{\text{ème}}$ élément du vecteur de projection $\mathbf{w}_f^i$, $\delta$ est le symbole de Kronecker, et $\Omega_{i,f}$ est l'ensemble des indices correspondant aux éléments pénalisables, étant compris que $Q_{i,f+1} \subset \Omega_{i,f}$ puisque les éléments non-pénalisés d'un projecteur à une itération $f$ ne sont pas pénalisables à l'itération $f$+1.

On comprendra notamment que la norme de l'expression (7-1) donne la somme des éléments non nuls dont les indices appartiennent à $\Omega_{i,f}$. Ainsi par exemple, dans ce cas, la pénalisation sera d'autant plus forte que le nombre d'éléments dont les indices appartiennent à $\Omega_{i,f}$ est plus élevé.

Afin de simplifier le calcul de minimisation dans les expressions (6-1), (6-2), (6-3), on pourra avantageusement opérer élément par élément, à savoir :

$$\min_{w_{f,k}^1}\left(\left\|\mathbf{z}_1^k - w_{f,k}^1\left(\mathbf{w}_f^3 \otimes \mathbf{w}_f^2\right)^T\right\|^2 + \lambda_1 g_q\left(w_{f,k}^1\right)\right), \ k = 1,\ldots,I_1 \tag{8-1}$$

$$\min_{w_{f,k}^2}\left(\left\|\mathbf{z}_2^k - w_{f,k}^2\left(\mathbf{w}_f^3 \otimes \mathbf{w}_f^1\right)^T\right\|^2 + \lambda_2 g_q\left(w_{f,k}^2\right)\right), \quad k = 1,\ldots,I_2 \tag{8-2}$$

$$\min_{w_{f,k}^3}\left(\left\|\mathbf{z}_3^k - w_{f,k}^3\left(\mathbf{w}_f^2 \otimes \mathbf{w}_f^1\right)^T\right\|^2 + \lambda_3 g_q\left(w_{f,k}^3\right)\right), \ k = 1,\ldots,I_3 \tag{8-3}$$

où les vecteurs $\mathbf{z}_1^k$, $\mathbf{z}_2^k$, $\mathbf{z}_3^k$ sont les $k^{\text{èmes}}$ vecteurs colonnes des matrices $\underline{\mathbf{XY}}_{u(1)}$, $\underline{\mathbf{XY}}_{u(2)}$, $\underline{\mathbf{XY}}_{u(3)}$ obtenues par déploiement du tenseur de covariance croisée $\underline{\mathbf{XY}}_u$ selon les modes 1, 2 et 3, respectivement.

Les fonctions $g_q$, $q = 0, \dfrac{1}{2}, 1$ s'appliquent à des valeurs scalaires et sont définies par :

$$g_0(w_{f,k}^i) = 1 - \delta_{0,w_{f,k}^i} \quad \text{si} \quad k \in \Omega_{i,f} \quad \text{et} \quad g_0(w_{f,k}^i) = 0 \quad \text{sinon} \tag{9-1}$$

$$g_{1/2}(w_{f,k}^i) = \sqrt{\left| w_{f,k}^i \right|} \quad \text{si} \quad k \in \Omega_{i,f} \quad \text{et} \quad g_{1/2}(w_{f,k}^i) = 0 \quad \text{sinon} \tag{9-2}$$

$$g_1(w_{f,k}^i) = \left| w_{f,k}^i \right| \quad \text{si} \quad k \in \Omega_{i,f} \quad \text{et} \quad g_1(w_{f,k}^i) = 0 \quad \text{sinon} \tag{9-3}$$

On peut montrer que le résultat de l'opération de minimisation pénalisée selon (8-1), (8-2), (8-3) pour la norme $L_0$ (c'est-à-dire $q=0$ ) donne :

$$\left( w_{f,k}^i \right)_{L0} = 0 \quad \text{si} \quad k \in \Omega_{i,f} \quad \text{et} \quad \left| \left( w_{f,k}^i \right)_{LS} \right| \leq Th_{L0}^i \tag{10-1}$$

$$\left( w_{f,k}^i \right)_{L0} = \left( w_{f,k}^i \right)_{LS} \quad \text{sinon} \tag{10-2}$$

où $\left( w_{f,k}^i \right)_{LS}$ représente la solution de minimisation au sens des moindres carrés (5-1), (5-2), (5-3), c'est-à-dire :

$$\left( w_{f,k}^1 \right)_{LS} = \frac{\mathbf{z}_1^k \left( \mathbf{w}_f^3 \otimes \mathbf{w}_f^2 \right)}{\left\| \mathbf{w}_f^3 \otimes \mathbf{w}_f^2 \right\|^2} \tag{11-1}$$

$$\left( w_{f,k}^2 \right)_{LS} = \frac{\mathbf{z}_2^k \left( \mathbf{w}_f^3 \otimes \mathbf{w}_f^1 \right)}{\left\| \mathbf{w}_f^3 \otimes \mathbf{w}_f^1 \right\|^2} \tag{11-2}$$

$$\left( w_{f,k}^3 \right)_{LS} = \frac{\mathbf{z}_3^k \left( \mathbf{w}_f^2 \otimes \mathbf{w}_f^1 \right)}{\left\| \mathbf{w}_f^2 \otimes \mathbf{w}_f^1 \right\|^2} \tag{11-3}$$

et les seuils $Th_{L0}^i$, $i=1,2,3$ sont donnés par :

$$Th_{L0}^{1} = \frac{\sqrt{\lambda_1}}{\left\| \mathbf{w}_f^3 \otimes \mathbf{w}_f^2 \right\|} \tag{12-1}$$

$$Th_{L0}^{2} = \frac{\sqrt{\lambda_2}}{\left\| \mathbf{w}_f^3 \otimes \mathbf{w}_f^1 \right\|} \tag{12-2}$$

$$Th_{L0}^{3} = \frac{\sqrt{\lambda_3}}{\left\| \mathbf{w}_f^2 \otimes \mathbf{w}_f^1 \right\|} \tag{12-3}$$

L'opération de minimisation pénalisée revient par conséquent à effectuer un seuillage sur les éléments des projecteurs obtenus à chaque étape de la méthode ALS.

De manière similaire, on peut montrer que l'opération de minimisation pénalisée selon (8-1), (8-2), (8-3) pour la norme $L_{1/2}$ (c'est-à-dire $q=1/2$) a pour solution :

$$\left( w_{f,k}^i \right)_{L0} = 0 \quad \text{si} \quad k \in \Omega_{i,f} \quad \text{et} \quad \left| \left( w_{f,k}^i \right)_{LS} \right| \le Th_{L0,5}^i \tag{13-1}$$

$$\left( w_{f,k}^i \right)_{L0} = \arg\min\left( h_{0,5}(0), h_{0,5}\left( B^i.\left( w_{f,k}^i \right)_{LS} \right) \right) \quad \text{si} \quad k \in \Omega_{i,f} \quad \text{et} \quad \left| \left( w_{f,k}^i \right)_{LS} \right| > Th_{L0,5}^i \tag{13-2}$$

$$\left( w_{f,k}^i \right)_{L0} = \left( w_{f,k}^i \right)_{LS} \quad \text{sinon} \tag{13-3}$$

où $h_{0,5}\left( w_{f,k}^i \right) = \left\| \mathbf{z}_1^k - w_{f,k}^i \left( \mathbf{w}_f^3 \otimes \mathbf{w}_f^2 \right)^T \right\|^2 + \lambda_1 \sqrt{\left| w_{f,k}^i \right|}$ et les seuils $Th_{L0,5}^i$, $i$=1,2,3 sont donnés par :

$$Th_{L0,5}^{1} = \frac{3}{4}\left( \frac{\lambda_1}{\left\| \mathbf{w}_f^3 \otimes \mathbf{w}_f^2 \right\|^2} \right)^{\frac{2}{3}} \tag{14-1}$$

$$Th_{L0,5}^{2} = \frac{3}{4}\left( \frac{\lambda_2}{\left\| \mathbf{w}_f^3 \otimes \mathbf{w}_f^1 \right\|^2} \right)^{\frac{2}{3}} \tag{14-2}$$

$$Th_{L0,5}^{3} = \frac{3}{4}\left( \frac{\lambda_3}{\left\| \mathbf{w}_f^2 \otimes \mathbf{w}_f^1 \right\|^2} \right)^{\frac{2}{3}} \tag{14-3}$$

Les coefficients $B^i$, $i = 1,2,3$ sont définis comme les solutions respectives des cubiques $x(1-x)^2 = C_i$, $i$=1,2,3, avec:

$$C^1 = \frac{\lambda_1^2}{16\left\|\mathbf{w}_f^3 \otimes \mathbf{w}_f^2\right\|^4 \left(\left(w_{f,k}^1\right)_{LS}\right)^3} \tag{15-1}$$

$$C^2 = \frac{\lambda_2^2}{16\left\|\mathbf{w}_f^3 \otimes \mathbf{w}_f^1\right\|^4 \left(\left(w_{f,k}^2\right)_{LS}\right)^3} \tag{15-2}$$

$$C^3 = \frac{\lambda_3^2}{16\left\|\mathbf{w}_f^2 \otimes \mathbf{w}_f^1\right\|^4 \left(\left(w_{f,k}^3\right)_{LS}\right)^3} \tag{15-3}$$

Enfin, de manière similaire, on peut montrer que l'opération de minimisation pénalisée selon (8-1), (8-2), (8-3) pour la norme $L_1$ (c'est-à-dire $q = 1$) a pour solution :

$$\left(w_{f,k}^i\right)_{L1} = 0 \quad \text{si} \quad k \in \Omega_{i,f} \quad \text{et} \quad \left|\left(w_{f,k}^i\right)_{LS}\right| \le Th_{L1}^i \tag{16-1}$$

$$\left(w_{f,k}^i\right)_{L1} = \operatorname{sgn}\left(\left(w_{f,k}^i\right)_{LS}\right)\left(\left|\left(w_{f,k}^i\right)_{LS}\right| - Th_{L1}^i\right) \quad \text{si} \quad k \in \Omega_{i,f} \quad \text{et} \quad \left|\left(w_{f,k}^i\right)_{LS}\right| > Th_{L1}^i \tag{16-2}$$

$$\left(w_{f,k}^i\right)_{L0} = \left(w_{f,k}^i\right)_{LS} \quad \text{sinon} \tag{16-3}$$

où les seuils $Th_{L1}^i$, $i = 1,2,3$ sont donnés par :

$$Th_{L1}^1 = \frac{\lambda_1}{\left\|\mathbf{w}_f^3 \otimes \mathbf{w}_f^2\right\|} \tag{17-1}$$

$$Th_{L1}^2 = \frac{\lambda_2}{\left\|\mathbf{w}_f^3 \otimes \mathbf{w}_f^1\right\|} \tag{17-2}$$

$$Th_{L1}^3 = \frac{\lambda_3}{\left\|\mathbf{w}_f^2 \otimes \mathbf{w}_f^1\right\|} \tag{17-3}$$

On comprend que, quelle que soit la norme utilisée, la minimalisation pénalisée se réduit avantageusement à un simple seuillage des éléments d'un projecteur à chaque étape de la méthode ALS. Cette minimalisation pénalisée permet de promouvoir une rareté par blocs des coefficients non nuls dans le modèle prédictif (*blockwise sparsity*) et par conséquent de simplifier le calcul. Il est important de noter que celle-ci peut être réalisée en ligne dans le but de réaliser une calibration incrémentale du modèle prédictif.

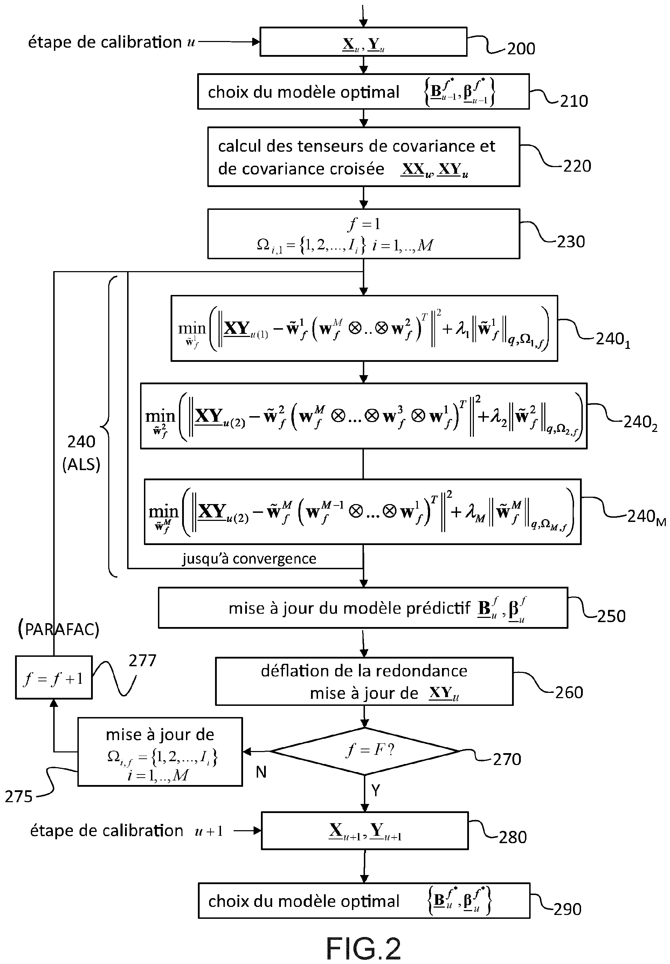

La Fig. 2 représente de manière schématique l'ordinogramme d'une méthode de calibration récursive d'une interface neuronale directe à régression multivariée pénalisée, selon un mode de réalisation de la présente invention.

A l'étape 200, on prend en compte de nouvelles données de calibration d'entrée et de sortie, respectivement sous la forme d'un tenseur d'entrée $\underline{\mathbf{X}}_u$ et d'un tenseur de sortie $\underline{\mathbf{Y}}_u$. Les données de calibration d'entrée résultent de la mesure des signaux électrophysiologiques. Les données de calibration de sortie résultent par exemple de la mesure de paramètres cinématiques de la trajectoire ou de signaux de commande d'effecteurs. Les tenseurs d'entrée et de sortie peuvent faire le cas échéant l'objet d'un prétraitement visant à les centrer et les normaliser, de manière connue en soi.

A l'étape 210, on détermine parmi l'ensemble des modèles prédictifs $\underline{\mathbf{B}}_{u-1}^{f}$, $\underline{\boldsymbol{\beta}}_{u-1}^{f}$, $f$ = 1,...,$F$ obtenus à l'étape précédente, le modèle prédictif $\left\{\underline{\mathbf{B}}_{u-1}^{f^*}, \underline{\boldsymbol{\beta}}_{u-1}^{f^*}\right\}$ correspondant à l'erreur de prédiction la plus faible. Ce modèle est utilisé pour calculer le tenseur de commande (tenseur de sortie) à partir du tenseur d'observation (tenseur d'entrée).

A l'étape 220, on met à jour les tenseurs de covariance $\underline{\mathbf{XX}}_u$ et de covariance croisée, $\underline{\mathbf{XY}}_u$, selon les relations (1-1) et (1-2), en tenant compte du facteur d'oubli.

On entre ensuite dans une première boucle itérative NPLS, dans laquelle on itère sur la dimension $f$ de l'espace des variables latentes, avec $f$ = 1,...,$F$ où F est une dimension maximale. Cette boucle itérative permet de générer successivement une pluralité de modèles prédictifs de l'interface neuronale directe, chaque modèle prédictif étant défini par un tenseur de coefficients de prédiction $\underline{\mathbf{B}}_u^f \in \mathbb{R}^{(I_1 \times ... \times I_N) \times (J_1 \times ... \times J_M)}$ et un tenseur de biais $\underline{\boldsymbol{\beta}}_u^f \in \mathbb{R}^{(J_1 \times ... \times J_M)}$.

A l'étape 230, on initialise $f$ avec $f$ = 1 et les ensembles $\Omega_{i,1}$, $i$ = 1,..,$M$ avec $\Omega_{i,1}$ = {1,2,...,$I_i$} pour $i$=1,..,$M$.

Pour chaque valeur de $f$, on effectue une décomposition PARAFAC du tenseur de covariance croisée à l'aide d'une méthode des moindres carrés alternés. A la différence d'une décomposition PARAFAC conventionnelle (cf. expressions (4-1), (4-2), (4-3)) utilisée dans la méthode REW-NPLS, on utilise ici une décomposition PARAFAC régularisée au moyen d'une métrique pénalisée, le terme de pénalisation visant à promouvoir une rareté par blocs des coefficients non nuls au sein du tenseur représentatif du modèle prédictif.

Plus précisément, en 240, pour chaque valeur de $f$, on recherche le $M$-uplet de projecteurs $\mathbf{w}_f^i$, $i$ = 1,...,$M$ de dimensions respectives $I_1, I_2,..,I_M$ permettant de minimiser ladite métrique pénalisée, comme exprimé en (6-1),(6-2),(6-3) dans le cas $M$ = 3. Cette minimisation se fait séquentiellement par ALS, projecteur par projecteur en $240_1$,...,$240_M$, les autres projecteurs étant considérés comme constants.

Avantageusement, à chacune de ces étapes, la minimisation porte sur les éléments du projecteur en question, comme décrit en relation avec les expressions (8-1) à (8-3) et (9-1) à (9-3).

Plus précisément, à chaque étape, les éléments du projecteur permettant de minimiser la métrique pénalisée sont obtenus au moyen d'un seuillage des éléments du projecteur minimisant la métrique non pénalisée, comme décrit en relation avec les expressions (10-1),(10-2) ; (13-1) à (13-3) ; (16-1) à (16-3).

Il est important de noter qu'à chaque itération $f$, seuls les éléments pénalisables sont pris en compte pour la minimisation de la métrique. A cet effet, on élimine de l'ensemble des indices pénalisables $\Omega_{i,f}$, les indices $k \in \Omega_{i,f}$ pour lesquels les éléments $w_{f,k}^i$ n'ont pas été pénalisée à l'itération $f$, pour générer l'ensemble des indices pénalisables relatif à l'itération suivante, $\Omega_{i,f+1}$.

Le tenseur du modèle prédictif, $\underline{\mathbf{B}}_u^f$, est obtenu à partir des projecteurs $\mathbf{w}_\varphi^i$, $i$ =1,...,$M$, $\varphi$=1,..,$f$ comme dans la méthode de régression REW-NPLS conventionnelle.

On trouvera le détail du calcul de $\underline{\mathbf{B}}_u^f$ à partir des projecteurs en question dans l'annexe A de l'article de A. Eliseyev *et al.* publié en 2017, cité dans la partie introductive. Les éléments du modèle prédictif $\underline{\mathbf{B}}_u^f$ correspondant aux indices pour lesquels les éléments $w_{f,k}^i$ ont été pénalisés, sont mis à zéro en 250.

Après chaque itération $f$, on élimine en 260 de $\underline{\mathbf{XX}}_u$ et $\underline{\mathbf{XY}}_u$ l'information redondante déjà prise en compte dans la régression sur l'espace de variables latentes, aux itérations précédentes 1,...,$f$. Le tenseur de covariance croisée $\underline{\mathbf{XY}}_u$ dans lequel cette redondance a été éliminée est utilisé pour la nouvelle itération de décomposition PARAFAC. L'étape 260 est connue sous le nom de déflation dans la régression NPLS.

Au terme des F itérations, en 270, on dispose par conséquent de F modèles prédictifs, $\underline{\mathbf{B}}_u^f$, $\underline{\boldsymbol{\beta}}_u^f$, $f$ = 1,...,$F$.

Après l'acquisition de nouveaux blocs de données de calibration d'entrée, $\underline{\mathbf{X}}_{u+1}$ et de données de calibration

de sortie, $\underline{\mathbf{Y}}_{u+1}$ à l'étape de calibration suivante, 280, on détermine en 290, le modèle prédictif optimal, $\left\{\underline{\mathbf{B}}_u^{f^*}, \underline{\boldsymbol{\beta}}_u^{f^*}\right\}$.

Ce modèle prédictif est utilisé pour calculer le tenseur de commande à partir du tenseur d'observation, jusqu'à l'étape de calibration suivante, $u+2$.

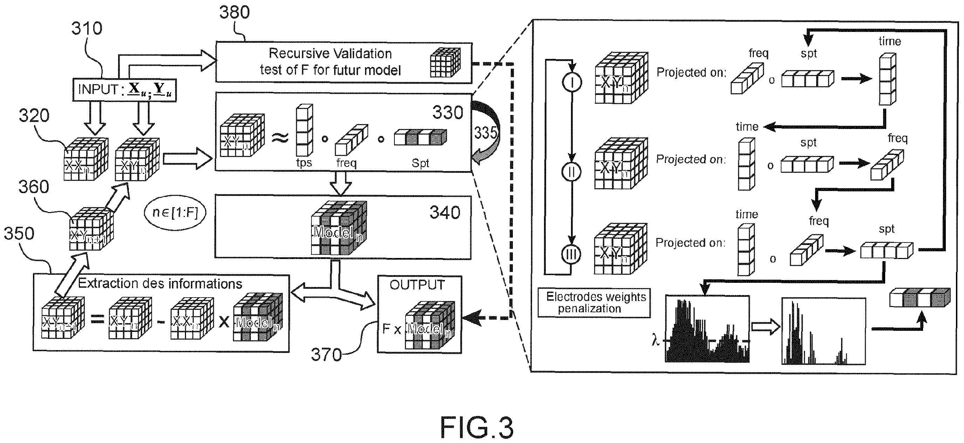

La Fig. 3 représente de manière schématique le mécanisme de mise à jour du modèle prédictif dans la méthode de calibration de la Fig. 2.

On a représenté en 310 la prise en compte d'un nouveau bloc de données de calibration d'entrée, représenté par le tenseur $\underline{\mathbf{X}}_u$ et d'un nouveau bloc de calibration de sortie, représenté par le tenseur $\underline{\mathbf{Y}}_u$.

Les tenseurs de covariance $\underline{\mathbf{XX}}_u$ et de covariance croisée, $\underline{\mathbf{XY}}_u$, en sont déduits en 320.

Pour chaque valeur de $f$, une décomposition PARAFAC du tenseur de covariance croisée est effectuée à l'aide d'une méthode des moindres carrés alternés (ALS) en 330, chaque étape de la décomposition PARAFAC portant sur un projecteur, cette décomposition étant régularisée au moyen d'un terme de pénalisation portant sur ce projecteur. La régularisation se traduit par un seuillage de certains éléments du projecteur obtenu par la méthode des moindres carrés conventionnelle, c'est-à-dire non pénalisée.

A chaque itération PARAFAC représentée par la boucle 335, on en déduit un modèle prédictif $\left\{\underline{\mathbf{B}}_u^f, \underline{\boldsymbol{\beta}}_u^f\right\}$ en 340.

Pour chaque modèle prédictif, on élimine en 350 l'information redondante dans le tenseur de covariance croisée (déflation) et l'on effectue une nouvelle itération PARAFAC sur le tenseur de covariance $\underline{\mathbf{XX}}_u$ et le nouveau tenseur de covariance croisée $\underline{\mathbf{XY}}_u$ (noté $\mathbf{XY}_{n+1}$ sur la figure) déflaté de ladite redondance, 360.

La boucle de validation récursive est représentée en 380. Elle permet de déterminer parmi les modèles prédictifs $\left\{\underline{\mathbf{B}}_u^f, \underline{\boldsymbol{\beta}}_u^f\right\}$, $f = 1,...,F,$ obtenus à l'étape de calibration précédente, celui qui minimise l'erreur de prédiction sur les nouveaux blocs de données de calibration d'entrée, $\underline{\mathbf{X}}_{u+1}$, et de sortie, $\underline{\mathbf{Y}}_{u+1}$.

## Revendications

1. Méthode de calibration en ligne d'une interface neuronale directe destinée à recevoir une pluralité de signaux électrophysiologiques pendant une pluralité de fenêtres d'observation associées à des instants d'observation, pour former un tenseur d'entrée et en déduire, au moyen d'un modèle prédictif, un tenseur de sortie fournissant des signaux de commande destinés à commander un ou plusieurs effecteurs, ladite calibration est effectuée lors d'une pluralité d'étapes de calibration, chaque étape de calibration u étant réalisée à partir de données de calibration d'entrée, représentées par un tenseur de calibration d'entrée, $\underline{\mathbf{X}}_u \in \mathbb{R}^{(I_1 \times ... \times I_N)}$, et de données de calibration de sortie représentées par un tenseur de calibration de sortie $\underline{\mathbf{Y}}_u \in \mathbb{R}^{(J_1 \times ... \times J_M)}$, ladite étape de calibration mettant en œuvre une régression REW-NPLS comportant :

   le calcul (220) d'un tenseur de covariance $\underline{\mathbf{XX}}_u$ à partir d'un tenseur de covariance $\underline{\mathbf{XX}}_{u-1}$ obtenu lors d'une étape de calibration précédente et du tenseur $\underline{\mathbf{X}}_u$, ainsi que le calcul d'un tenseur de covariance croisée $\underline{\mathbf{XY}}_u$ à partir d'un tenseur de covariance croisée $\underline{\mathbf{XY}}_{u-1}$ obtenu lors de ladite étape de calibration précédente et des tenseurs $\underline{\mathbf{X}}_u$ et $\underline{\mathbf{Y}}_u$ ;
   une décomposition PARAFAC itérative du tenseur de covariance croisée $\underline{\mathbf{XY}}_u$, chaque itération PARAFAC étant associée à une dimension $f$ de l'espace des variables latentes sur lequel est effectuée ladite régression, chaque itération fournissant une pluralité de projecteurs $\mathbf{w}_f^i, i = 1,..,M$ de tailles respectives $I_1, I_2,...I_M$ et un modèle prédictif défini par un tenseur de coefficients de prédiction $\underline{\mathbf{B}}_u^f \in \mathbb{R}^{(I_1 \times ... \times I_N) \times (J_1 \times ... \times J_M)}$ et un tenseur de biais $\underline{\boldsymbol{\beta}}_u^f \in \mathbb{R}^{(J_1 \times ... \times J_M)}$ ;
   **caractérisée en ce que** chaque itération PARAFAC comprend une séquence de $M$ étapes élémentaires ($240_1$, $240_1$,.. $240_M$) de minimisation d'une métrique selon la méthode des moindres carrés alternés, chaque étape élémentaire de minimisation portant sur un projecteur et considérant les autres comme constants, ladite métrique comportant un terme de pénalisation fonction de la norme de ce projecteur, les éléments de ce projecteur ne faisant pas l'objet d'une pénalisation lors d'une itération PARAFAC $f$ n'étant pas pénalisables lors des itérations PARAFAC suivantes.

2. Méthode de calibration en ligne d'une interface neuronale directe selon la revendication 1, **caractérisée en ce qu'**une étape élémentaire de minimisation portant sur un projecteur $\mathbf{w}_f^i$ vise à minimiser la métrique pénalisée

$$\min_{\tilde{\mathbf{w}}_f^i}\left(\left\|\underline{\mathbf{XY}}_{u(i)} - \tilde{\mathbf{w}}_f^i\left(\mathbf{w}_f^M \otimes ... \mathbf{w}_f^{i+1} \otimes \mathbf{w}_f^{i-1} \otimes ... \otimes \mathbf{w}_f^1\right)^T\right\|^2 + \lambda_i \left\|\tilde{\mathbf{w}}_f^i\right\|_{q,\Omega_{i,f}}\right)$$ où $\tilde{\mathbf{w}}_f^i$ est un projecteur non

normalisé et $\mathbf{w}_f^m = \dfrac{\tilde{\mathbf{w}}_f^m}{\left\|\tilde{\mathbf{w}}_f^m\right\|}$ , $m=1,...,M; m \neq i$ sont des projecteurs normalisés, $\underline{\mathbf{XY}}_{u(i)}$ est une matrice de taille

$I_i \times \displaystyle\prod_{\substack{n=1 \\ n\neq i}}^{N} I_n$ obtenue par déploiement du tenseur de covariance croisée $\underline{\mathbf{XY}}_u$ en prenant le mode $i$ comme référence,

$\otimes$ est le produit de Kronecker, $\lambda_i$ est un coefficient strictement positif, $\|\mathbf{w}\|_{q,\Omega_{i,f}}$ , $q = 0, \dfrac{1}{2}, 1$ est une norme définie

par $\left\|\mathbf{w}_f^i\right\|_{0,\Omega_{i,f},} = \displaystyle\sum_{k\in\Omega_{i,f}}\left(1 - \delta_{0,w_{f,k}^i}\right)$ , $\left\|\mathbf{w}_f^i\right\|_{1/2,\Omega_{i,f}} = \displaystyle\sum_{k\in\Omega_{i,f}}\sqrt{\left|w_{f,k}^i\right|}$ , $\left\|\mathbf{w}_f^i\right\|_{1,\Omega_{i,f}} = \displaystyle\sum_{k\in\Omega_{i,f}}\left|w_{f,k}^i\right|$ où $w_{f,k}^i$ est le

$k^{ème}$ élément du projecteur $\mathbf{w}_f^i$ , $\delta$ est le symbole de Kronecker, et $\Omega_{i,f}$ est l'ensemble des indices correspondant aux éléments pénalisables du projecteur $\mathbf{w}_f^i$ à l'itération PARAFAC $f$ .

3. Méthode de calibration en ligne d'une interface neuronale directe selon la revendication 1, **caractérisée en ce qu'**une étape élémentaire de minimisation portant sur un projecteur $\mathbf{w}_f^i$ vise à minimiser les métriques pénalisées

$$\min_{w_{f,k}^i}\left(\left\|\mathbf{z}_i^k - w_{f,k}^i\left(\left(\mathbf{w}_f^M \otimes ... \mathbf{w}_f^{i+1} \otimes \mathbf{w}_f^{i-1} \otimes ... \otimes \mathbf{w}_f^1\right)^T\right)^T\right\|^2 + \lambda_i g_q\left(w_{f,k}^i\right)\right)$$ , $k=1,...,I_i$ où $\mathbf{z}_i^k$ est le kème vecteur colonne de la matrice $\underline{\mathbf{XY}}_{u(i)}$ de taille $I_i \times \displaystyle\prod_{\substack{n=1 \\ n\neq i}}^{N} I_n$ obtenue par déploiement du tenseur de covariance croisée $\underline{\mathbf{XY}}_u$ en prenant le mode $i$ comme référence, $\otimes$ est le produit de Kronecker, $\lambda_i$ est un coefficient strictement positif, et les fonctions $g_q$, $q = 0, \dfrac{1}{2}, 1$ sont définies par :

$$g_0(w_{f,k}^i) = 1 - \delta_{0,w_{f,k}^i} \quad \text{si } k \in \Omega_{i,f} \text{ et } g_0(w_{f,k}^i) = 0 \text{ sinon}$$

$$g_{1/2}(w_{f,k}^i) = \sqrt{\left|w_{f,k}^i\right|} \quad \text{si } k \in \Omega_{i,f} \text{ et } g_{1/2}(w_{f,k}^i) = 0 \text{ sinon}$$

$$g_1(w^i_{f,k}) = \left| w^i_{f,k} \right| \quad \text{si} \quad k \in \Omega_{i,f} \quad \text{et} \quad g_1(w^i_{f,k}) = 0 \quad \text{sinon}$$

où $\Omega_{i,f}$ est l'ensemble des indices $k$ correspondant aux éléments $w^i_{f,k}$ pénalisables du projecteur $\mathbf{w}^i_f$ à l'itération PARAFAC $f$.

**4.** Méthode de calibration en ligne d'une interface neuronale directe selon la revendication 3, **caractérisée en ce que** $q = 0$ et que les éléments $w^i_{f,k}$ résultant de la minimisation sont donnés par :

$$\left( w^i_{f,k} \right)_{L0} = 0 \quad \text{si} \quad k \in \Omega_{i,f} \quad \text{et} \quad \left| \left( w^i_{f,k} \right)_{LS} \right| \leq Th^i_{L0}$$

$$\left( w^i_{f,k} \right)_{L0} = \left( w^i_{f,k} \right)_{LS} \quad \text{sinon}$$

où $\left( w^i_{f,k} \right)_{LS}$ représente la solution de minimisation au sens des moindres carrés :

$$\left( w^i_{f,k} \right)_{LS} = \frac{\mathbf{z}^k_i \left( \mathbf{w}^M_f \otimes \ldots \mathbf{w}^{i+1}_f \otimes \mathbf{w}^{i-1}_f \otimes \ldots \otimes \mathbf{w}^1_f \right)}{\left\| \mathbf{w}^M_f \otimes \ldots \mathbf{w}^{i+1}_f \otimes \mathbf{w}^{i-1}_f \otimes \ldots \otimes \mathbf{w}^1_f \right\|^2}$$

et que le seuil $Th^i_{L0}$ est donné par $\displaystyle Th^i_{L0} = \frac{\sqrt{\lambda_i}}{\left\| \mathbf{w}^M_f \otimes \ldots \mathbf{w}^{i+1}_f \otimes \mathbf{w}^{i-1}_f \otimes \ldots \otimes \mathbf{w}^1_f \right\|}$ .

**5.** Méthode de calibration en ligne d'une interface neuronale directe selon la revendication 3, **caractérisée en ce que** $q = \dfrac{1}{2}$ et que les éléments $w^i_{f,k}$ résultant de la minimisation sont donnés par :

$$\left( w^i_{f,k} \right)_{L0} = 0 \quad \text{si} \quad k \in \Omega_{i,f} \quad \text{et} \quad \left| \left( w^i_{f,k} \right)_{LS} \right| \leq Th^i_{L0,5}$$

$$\left( w^i_{f,k} \right)_{L0} = \arg\min \left( h_{0,5}(0), h_{0,5}\left( B^i \cdot \left( w^i_{f,k} \right)_{LS} \right) \right) \quad \text{si} \quad k \in \Omega_{i,f} \quad \text{et} \quad \left| \left( w^i_{f,k} \right)_{LS} \right| > Th^i_{L0,5}$$

$$\left( w^i_{f,k} \right)_{L0} = \left( w^i_{f,k} \right)_{LS} \quad \text{sinon}$$

où $h_{0,5}\left( w^i_{f,k} \right) = \left\| \mathbf{z}^k_1 - w^i_{f,k} \left( \mathbf{w}^3_f \otimes \mathbf{w}^2_f \right)^T \right\|^2 + \lambda_i \sqrt{\left| w^i_{f,k} \right|}$ et où $\left( w^i_{f,k} \right)_{LS}$ représente la solution de minimisation au sens des moindres carrés :

$$\left(w_{f,k}^i\right)_{LS} = \frac{\mathbf{z}_i^k \left(\mathbf{w}_f^M \otimes ... \mathbf{w}_f^{i+1} \otimes \mathbf{w}_f^{i-1} \otimes ... \otimes \mathbf{w}_f^1\right)}{\left\|\mathbf{w}_f^M \otimes ... \mathbf{w}_f^{i+1} \otimes \mathbf{w}_f^{i-1} \otimes ... \otimes \mathbf{w}_f^1\right\|^2}$$

et le seuil $Th_{L0,5}^i$ est donné par

$$Th_{L0,5}^i = \frac{3}{4}\left(\frac{\lambda_i}{\left\|\mathbf{w}_f^M \otimes ... \mathbf{w}_f^{i+1} \otimes \mathbf{w}_f^{i-1} \otimes ... \otimes \mathbf{w}_f^1\right\|^2}\right)^{\frac{2}{3}}$$

.

**6.** Méthode de calibration en ligne d'une interface neuronale directe selon la revendication 3, **caractérisée en ce que** $q = 1$ et que les éléments $w_{f,k}^i$ résultant de la minimisation sont donnés par :

$$\left(w_{f,k}^i\right)_{L1} = 0 \quad \text{si} \quad k \in \Omega_{i,f} \quad \text{et} \quad \left|\left(w_{f,k}^i\right)_{LS}\right| \le Th_{L1}^i$$

$$\left(w_{f,k}^i\right)_{L1} = \text{sgn}\left(\left(w_{f,k}^i\right)_{LS}\right)\left(\left|\left(w_{f,k}^i\right)_{LS}\right| - Th_{L1}^i\right) \quad \text{si} \quad k \in \Omega_{i,f} \text{ et } \left|\left(w_{f,k}^i\right)_{LS}\right| > Th_{L1}^i$$

$$\left(w_{f,k}^i\right)_{L0} = \left(w_{f,k}^i\right)_{LS} \quad \text{sinon}$$

où $\left(w_{f,k}^i\right)_{LS}$ représente la solution de minimisation au sens des moindres carrés :

$$\left(w_{f,k}^i\right)_{LS} = \frac{\mathbf{z}_i^k \left(\mathbf{w}_f^M \otimes ... \mathbf{w}_f^{i+1} \otimes \mathbf{w}_f^{i-1} \otimes ... \otimes \mathbf{w}_f^1\right)}{\left\|\mathbf{w}_f^M \otimes ... \mathbf{w}_f^{i+1} \otimes \mathbf{w}_f^{i-1} \otimes ... \otimes \mathbf{w}_f^1\right\|^2}$$

et le seuil $Th_{L1}^i$ est donné par

$$Th_{L1}^i = \frac{\lambda_i}{\left\|\mathbf{w}_f^M \otimes ... \mathbf{w}_f^{i+1} \otimes \mathbf{w}_f^{i-1} \otimes ... \otimes \mathbf{w}_f^1\right\|}$$

**7.** Méthode de calibration en ligne d'une interface neuronale directe selon l'une des revendications précédentes, **caractérisée en ce que** les signaux électrophysiologiques sont des signaux ECoG.

## FIG.1A

## FIG.1B

## FIG.1C

FIG.2

FIG.3

# EP 3 985 530 A1

Europäisches
Patentamt

European
Patent Office

Office européen
des brevets

## RAPPORT DE RECHERCHE EUROPEENNE

Numéro de la demande

**EP 21 20 1998**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y,D | ANDREY ELISEYEV ET AL: "Recursive Exponentially Weighted N-way Partial Least Squares Regression with Recursive-Validation of Hyper-Parameters in Brain-Computer Interface Applications", NATURE, SCIENTIFIC REPORTS, vol. 7, no. 1, 24 novembre 2017 (2017-11-24), XP055705682, DOI: 10.1038/s41598-017-16579-9 * le document en entier * | 1-7 | INV. G06F17/16 A61F2/72 A61F4/00 |
| Y | CICHOCKI A ET AL: "Fast Local Algorithms for Large Scale Nonnegative Matrix and Tensor Factorizations", IEICE TRANSACTIONS ON FUNDAMENTALS OF ELECTRONICS,COMMUNICATIONS AND COMPUTER SCIENCES, ENGINEERING SCIENCES SOCIETY, TOKYO, JP, vol. E92A, no. 3, 1 mars 2009 (2009-03-01) , pages 708-721, XP001544993, ISSN: 0916-8508, DOI: 10.1587/TRANSFUN.E92.A.708 * section 3 * | 1-7 | |
| A | WO 2011/144959 A1 (COMMISSARIAT ENERGIE ATOMIQUE [FR]; AKSENOVA TETIANA [FR] ET AL.) 24 novembre 2011 (2011-11-24) * le document en entier * | 1-7 | DOMAINES TECHNIQUES RECHERCHES (IPC) G06F A61F |
| A,D | ANDREY ELISEYEV ET AL: "Recursive N-Way Partial Least Squares for Brain-Computer Interface", PLOS ONE, vol. 8, no. 7, 26 juillet 2013 (2013-07-26), XP055178425, DOI: 10.1371/journal.pone.0069962 * le document en entier * | 1-7 | |

-/--

Le présent rapport a été établi pour toutes les revendications

| Lieu de la recherche | Date d'achèvement de la recherche | Examinateur |
|---|---|---|
| Berlin | 28 février 2022 | Virnik, Elena |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................................................
& : membre de la même famille, document correspondant

EPO FORM 1503 03.82 (P04C02)

page 1 de 2

| Europäisches Patentamt / European Patent Office / Office européen des brevets | **RAPPORT DE RECHERCHE EUROPEENNE** | Numéro de la demande  EP 21 20 1998 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | Kimura Keigo: "A Study on Efficient Algorithms for Nonnegative Matrix/Tensor Factorization", Doctoral Dissertation, 23 mars 2017 (2017-03-23), pages 1-11, XP055819105, United States DOI: 10.1016/j.ahj.2021.01.002 Extrait de l'Internet: URL:https://eprints.lib.hokudai.ac.jp/dspace/bitstream/2115/65379/1/Keigo_Kimura.pdf [extrait le 2021-06-29] * section 5 * ----- | 1-7 | |
| | | | **DOMAINES TECHNIQUES RECHERCHES (IPC)** |

Le présent rapport a été établi pour toutes les revendications

| Lieu de la recherche  **Berlin** | Date d'achèvement de la recherche  **28 février 2022** | Examinateur  **Virnik, Elena** |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 21 20 1998

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-02-2022

| Document brevet cité au rapport de recherche | Date de publication | Membre(s) de la famille de brevet(s) | | Date de publication |
|---|---|---|---|---|
| WO 2011144959 A1 | 24-11-2011 | EP | 2571461 A1 | 27-03-2013 |
| | | US | 2013165812 A1 | 27-06-2013 |
| | | WO | 2011144959 A1 | 24-11-2011 |

EPO FORM P0460

Pour tout renseignement concernant cette annexe : voir Journal Officiel de l'Office européen des brevets, No.12/82

## RÉFÉRENCES CITÉES DANS LA DESCRIPTION

### Documents brevets cités dans la description

- FR 3061318 A
- FR 3046471 A

### Littérature non-brevet citée dans la description

- **A. ELISEYEV et al.** Recursive exponentially weighted N-way Partial Least Squares régression with recursive validation of hyper-parameters in Brain-Computer Interface applications. *Scientific Reports,* Novembre 2017, vol. 7 (1), 16281
- **A. ELISEYEV et al.** L1-penalized N-way PLS for subset of electrodes selection in BCI experiments. *J. Neural Eng.,* vol. 9 (4), 045010